# EUROPEAN PATENT APPLICATION

(11) **EP 3 569 615 A1**
(43) Date of publication of application: **20.11.2019**
(21) Application number: 17890966.9
(22) Date of filing: 13.01.2017
(51) Int. Cl.: C07K 16/00, C12N 15/09

(54) **METHOD FOR IMPROVING BINDING AFFINITY OF IGG ANTIBODY TO FCRN AND PROLONGING SERUM HALF-LIFE PERIOD THEREOF**

(71) Applicant: Hanx Biopharmaceutics, Inc, Wuhan, Hubei 430074 (CN)
(72) Inventor: ZHANG, Faming, Wuhan Hubei 430074 (CN); XI, Gan, Hangzhou Zhejiang 310051 (CN); HUANG, Ying, Hangzhou Zhejiang 310051 (CN)
(74) Representative: Pföstl, Andreas
(86) International application number: PCT/CN2017/071124
(87) International publication number: WO 2018/129713

(57) **Abstract**

Provided is a method for improving the binding affinity of an IgG antibody to FcRn and prolonging a serum half-life period thereof. An objective of the method is to allow amino acids at No. 254, 308 and 434 sites in FcRn binding site regions in a heavy chain constant region of the IgG antibody to have mutations.

SEQUENCE LISTING

<110>  HANX BIOPHARMACEUTICS, INC

<120>  METHOD FOR INCREASING BINDING AFFINITY OF IGG-LIKE ANTIBODY TO FCRN
AND PROLONGING SERUM HALF-LIFE THEREOF

<130>  25623-WOEP

<160>  4

<170>  PatentIn version 3.3

<210>  1
<211>  445
<212>  PRT
<213>  Artificial

<220>
<223>  amino acid sequence of  heavy chain of H8L2

<400>  1

Glu Val Gln Leu Val Gln Ser Gly Gly Gly Leu Val Gln Pro Gly Gly
1               5                   10                  15

Ser Leu Lys Leu Ser Cys Ala Ala Ser Gly Phe Thr Phe Ser Ser Tyr
            20                  25                  30

Gly Met Ser Trp Val Arg Gln Ala Pro Gly Lys Gly Leu Asp Trp Val
        35                  40                  45

Ala Thr Ile Ser Gly Gly Gly Arg Asp Thr Tyr Tyr Pro Asp Ser Val
    50                  55                  60

Lys Gly Arg Phe Thr Ile Ser Arg Asp Asn Ser Lys Asn Asn Leu Tyr
65                  70                  75                  80

Leu Gln Met Asn Ser Leu Arg Ala Glu Asp Thr Ala Leu Tyr Tyr Cys
                85                  90                  95

Ala Arg Gln Lys Gly Glu Ala Trp Phe Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Thr Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Pro Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Arg Leu Thr Val Asp Lys Ser Arg Trp Gln
                405                 410                 415

Glu Gly Asn Val Phe Ser Cys Ser Val Met His Glu Ala Leu His Ala
            420                 425                 430

His Tyr Thr Gln Lys Ser Leu Ser Leu Ser Leu Gly Lys
        435                 440                 445

<210>  2
<211>  1392
<212>  DNA
<213>  Artificial

<220>
<223>  nucleotide sequence encoding  heavy chain of H8L2

<400>  2
atgggatgga gctgtatcat cctcttcttg gtagcaacag ctacaggcgc gcactccgag      60

gtgcagctgg tgcagtctgg cggcggactg gtgcagcccg gcgggtcact gaagctgagc     120

tgcgccgctt ccggcttcac ctttagctcc tacggaatgt cctgggtgcg acaggcaccc     180

gggaaggggc tggactgggt cgctactatc tcaggaggcg ggagagacac ctactatcct     240

gatagcgtca agggccggtt cacaattagc cgggacaaca gcaagaacaa tctgtacctg     300

cagatgaaca gcctgagggc tgaggatact gcactgtact attgtgcccg ccagaagggc     360

gaagcatggt ttgcctattg gggccaggga accctggtca ccgtctcctc agcttccacc     420

aagggcccat ccgtcttccc cctggcgccc tgctccagga gcacctccga gagcacagcc     480

gccctgggct gcctggtcaa ggactacttc cccgaaccgg tgacggtgtc gtggaactca     540

ggcgccctga ccagcggcgt gcacaccttc ccggctgtcc tacagtcctc aggactctac     600

tccctcagca gcgtggtgac tgtgccctcc agcagcttgg gcacgaagac ctacacctgc     660

aacgtagatc acaagcccag caacaccaag gtggacaaga gagttgagtc caaatatggt     720

cccccatgcc caccatgccc agcacctgag ttcctggggg gaccatcagt cttcctgttc     780

cccccaaaac ccaaggacac tctcatgatc tcccggaccc ctgaggtcac gtgcgtggtg     840

gtggacgtga gccaggaaga ccccgaggtc cagttcaact ggtacgtgga tggcgtggag     900

gtgcataatg ccaagacaaa gccgcgggag gagcagttca acagcacgta ccgtgtggtc     960

agcgtcctca ccgtcctgca ccaggactgg ctgaacggca aggagtacaa gtgcaaggtc    1020

tccaacaaag gcctcccgtc ctccatcgag aaaaccatct ccaaagccaa agggcagccc    1080

cgagagccac aggtgtacac cctgccccca tcccaggagg agatgaccaa gaaccaggtc    1140

agcctgacct gcctggtcaa aggcttctac cccagcgaca tcgccgtgga gtgggagagc    1200

aatgggcagc cggagaacaa ctacaagacc acgcctcccg tgctggactc cgacggctcc    1260

ttcttcctct acagcaggct aaccgtggac aagagcaggt ggcaggaggg gaatgtcttc    1320

tcatgctccg tgatgcatga ggctctgcac gcccactaca cacagaagag cctctccctg    1380

tctctgggta aa                                                          1392

<210>  3
<211>  218
<212>  PRT
<213>  Artificial

<220>
<223>  amino acid sequence of light chain of H8L2

<400>  3

Asp Ile Val Leu Thr Gln Ser Pro Ala Ser Leu Ala Val Ser Pro Gly
1               5                   10                  15

Gln Arg Ala Thr Ile Thr Cys Arg Ala Ser Glu Ser Val Asp Asn Tyr
            20                  25                  30

Gly Ile Ser Phe Met Asn Trp Phe Gln Gln Lys Pro Gly Gln Pro Pro
        35                  40                  45

Lys Leu Leu Ile Tyr Ala Ala Ser Asn Lys Gly Thr Gly Val Pro Ala
    50                  55                  60

Arg Phe Ser Gly Ser Gly Ser Gly Thr Asp Phe Thr Leu Asn Ile Asn
65                  70                  75                  80

Pro Met Glu Glu Asn Asp Thr Ala Met Tyr Phe Cys Gln Gln Ser Lys
                85                  90                  95

Glu Val Pro Trp Thr Phe Gly Gly Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro

195                 200                 205

Val Thr Lys Ser Phe Asn Arg Gly Glu Cys
    210                 215

<210>  4
<211>  711
<212>  DNA
<213>  Artificial

<220>
<223>  nucleotide sequence encoding  light chain of H8L2

<400>  4
atgggatgga gctgtatcat cctcttcttg gtagcaacag ctacaggcgt gcactccgat      60

attgtgctga ctcagagccc tgcttccctg gccgtgtctc caggacagcg agctaccatc     120

acatgcagag catctgagag tgtggacaac tacggaatta gtttcatgaa ttggtttcag     180

cagaagcccg gccagccccc taaactgctg atctatgccg ccagcaacaa gggcaccggg     240

gtgcctgctc gattctcagg aagcggctcc gggacagact ttactctgaa cattaaccca     300

atggaggaaa atgatacagc aatgtacttc tgccagcaga gcaaggaggt gccctggacc     360

tttggcgggg gaacaaagct ggaaatcaaa cgaactgtgg ctgcaccatc tgtcttcatc     420

ttcccgccat ctgatgagca gttgaaatct ggaactgcct ctgttgtgtg cctgctgaat     480

aacttctatc ccagagaggc caaagtacag tggaaggtgg ataacgccct ccaatcgggt     540

aactcccagg agagtgtcac agagcaggac agcaaggaca gcacctacag cctcagcagc     600

accctgacgc tgagcaaagc agactacgag aaacacaaag tctacgcctg cgaagtcacc     660

catcagggcc tgagctcgcc cgtcacaaag agcttcaaca ggggagagtg t              711

## Description

### PRIORITY

None

### FIELD

The present disclosure relates to the field of immunology and antibody engineering, in particular to a method for increasing binding affinity of an IgG-like antibody to FcRn and prolonging serum half-life thereof, and a modified IgG-like antibody.

### BACKGROUND

Antibody also known as immunoglobulin (Ig) is a protein that specifically binds to an antigen, which consists of four peptide chains, i.e. two heavy chains (H chain) in a large molecular weight of 50kD and two light chains (L chain) in a small molecular weight of 23kD which are linked by a disulfide bond. The region at the C-terminus of polypeptide having stable species, number and order of amino acids, is called as a constant region or stable region (i.e. C region), which accounts for 1/2 sequence of the L chain and 3/4 sequence of the H chain. The region at the N-terminus of polypeptide having different species and order of amino acids, is called as a variable region (i.e. V region), which determines the diversity of antibody due to the hyper-variability, and thus determines the specific binding between antibody and antigen.

Human being mainly contains five different types of antibodies, including IgA (further including IgA1 and IgA2 subclasses), IgD, IgE, IgG (including IgG1, IgG2, IgG3 and IgG4 subclasses) and IgM, where IgG is the most important immunoglobulin for human being, thus being commonly used in therapy. However, the natural IgG antibody exhibits severe deficiencies such as low persistence and short serum half-life in blood circulation, resulting in directly affecting the efficacy of therapy due to IgG clearance rate, and further generating side effect in patients and increasing the frequency and dosage of drug administration. Therefore, it is of great significance to increase binding affinity of the IgG-like antibody (especially natural IgG-like antibody) to FcRn and prolong serum half-life thereof.

However, there is still a need for a method of increasing binding affinity of an IgG-like antibody (especially a natural IgG-like antibody) to FcRn and prolonging serum half-life thereof.

### SUMMARY

Embodiments of the present disclosure aim at solving at least one of the problems existing in the related art to at least some extent. For this purpose, an object of the present disclosure is to provide a means for increasing binding affinity of an IgG-like antibody to FcRn and prolonging serum half-life thereof.

It should be noted that the present disclosure is accomplished by present inventors according to the following discoveries and work.

The IgG antibody can be hydrolyzed with papain by cleaving the disulfide bond at the N-terminus of a hinge region of the IgG antibody, thus obtaining three fragments, i.e. two identical fragments of antigen binding (i.e. Fab) and a fragment crystallizable (i.e. Fc). The crystallizable fragment Fc of antibody interacts with a variety of Fc receptors and ligands, thereby conferring important effector functions to the antibody, including initiation of complement-dependent cytotoxicity (CDC), phagocytosis and antibody-dependent cell-mediated cytotoxicity (ADCC), and transportation of antibody through cellular barrier via transcytosis. In addition, the Fc fragment is important for maintaining the serum half-life of IgG-like antibody.

Neonatal Fc receptor (FcRn) is a receptor responsible for active transportation of immunoglobulin G (IgG) by epithelial cells, which is a heterodimer consisting of an alpha chain subunit and a beta chain subunit which are linked with a non-covalent bond. The Fc fragment of IgG antibody includes two identical polypeptide chains, each of them binding to a single FcRn molecule through its individual FcRn binding site. For an adult mammal, the IgG antibody binds to FcRn through the Fc fragment thereby protecting the IgG antibody from degradation, thus the Fc fragment is of critical importance in maintaining serum antibody level. The IgG antibody binding to FcRn after endocytosed by endothelial cells will circulate in blood circulation, while the IgG antibody not binding to FcRn will be degraded by lysosomes. Thus, the Fc fragment is critical for binding affinity of the IgG antibody binds to FcRn.

Thus, the present inventors have attempted to propose a method for increasing binding affinity to FcRn and prolonging serum half-life of an IgG-like antibody (especially a natural IgG-like antibody) by changing the sequence of a heavy chain constant region of the IgG-like antibody (i.e. the sequence of Fc fragment). That is, the present inventors aim at improving the serum half-life and binding affinity to FcRn by mutating the amino acid in Fc fragment of IgG-like antibody. It is found in surprise by the present inventors after a series of experimental designs and researches that the binding affinity of the IgG-like antibody to FcRn can be improved by introducing amino acid mutations to the Fc fragment of human IgG-like antibody, thus increasing the serum half-life of the IgG-like antibody. In specific, the Fc fragment of the IgG-like antibody is mutated at amino acid positions 254, 308 and 434 with amino acids which are different from those in a wild-type IgG-like antibody (which contains no amino acid mutation), thus obtaining an optimized antibody which exhibits prolonged serum half-life compared to the wild-type IgG-like antibody. In other words, the present inventors found that mutating amino acids at positions 254, 308 and 434 in the FcRn-binding site of the heavy chain constant region of the IgG-like antibody is capable of effectively increasing binding affinity of the IgG-like antibody to FcRn and prolonging serum half-life thereof.

Thus, in one aspect, the present disclosure in embodiments provides a method for increasing binding affinity of an IgG-like antibody to FcRn and prolonging serum half-life thereof. In embodiments, the method includes mutating amino acids at positions 254, 308 and 434 in an FcRn-binding site of a heavy chain constant region of the IgG-like antibody. Thus, the modified IgG-like antibody obtained has effectively increased binding affinity to FcRn and prolonged serum half-life, while maintaining the binding affinity to a specific antigen.

In addition, it should be noted that the method of mutating the amino acids at positions 254, 308 and 434 in the FcRn-binding site of the heavy chain constant region of the IgG-like antibody is not particularly limited, as long as the amino acid mutations at positions 254, 308 and 434 are achievable, for example, chemical induction method, site-directed mutagenesis and the like. In general, the amino acid mutations can be obtained in gene level by mutating the nucleotides in the gene encoding the IgG-like antibody (corresponding to the amino acids at positions 254, 308 and 434) with specific nucleotides which corresponds to the mutated amino acids, thus obtaining an IgG-like antibody variant (also called as a modified IgG-like antibody herein) based on the encoding gene mutated. Thus, from this aspect, the method for increasing binding affinity of IgG-like antibody to FcRn and prolonging serum half-life thereof can be regarded as a method for preparing an IgG-like antibody variant which has increased binding affinity to FcRn and prolonged serum half-life compared to a known IgG-like antibody.

In embodiments, the amino acids at positions 254, 308 and 434 in the FcRn-binding site of the heavy chain constant region of the IgG-like antibody are mutated into threonine, proline and alanine respectively. Thus, the serum half-life of the IgG-like antibody is prolonged and the binding affinity of the IgG-like antibody to FcRn is increased significantly.

In another aspect, the present disclosure in embodiments provides a modified IgG-like antibody. In embodiments, the modified IgG-like antibody has amino acid mutations at positions 254, 308 and 434 in an FcRn-binding site of a heavy chain constant region with respect to a wild-type IgG-like antibody. It is found by the present inventors in surprise that the modified IgG-like antibody exhibits increased binding affinity to FcRn and prolonged serum half-life compared to the wild-type IgG-like antibody, while maintaining the binding affinity to a specific antigen.

In embodiments, the amino acid mutations at positions 254, 308 and 434 in the FcRn-binding site of the heavy chain constant region of the modified IgG-like antibody are respectively threonine, proline and alanine with respect to the wild-type IgG-like antibody. Thus, the modified IgG-like antibody exhibits prolonged serum half-life and significantly increased binding affinity to FcRn compared to the wild-type IgG-like antibody.

In a further aspect, the present disclosure in embodiments provides a method for preparing the modified IgG-like antibody described above. In embodiments, the method includes: generating a nucleic acid sequence encoding a target IgG-like antibody via gene synthesis according to an amino acid sequence of the target IgG-like antibody; constructing an expression vector comprising the nucleic acid sequence encoding the target IgG-like antibody; and transfecting an antibody producing cell with the expression vector, such that the antibody producing cell expresses and secretes the target IgG-like antibody, wherein the target IgG-like antibody is the modified IgG-like antibody.

Therefore, the modified IgG-like antibody (i.e. the target IgG-like antibody) which exhibits increased binding affinity to FcRn and prolonged serum half-life relative to the wild-type IgG-like antibody can be prepared in an easy and effective way. In other words, according to embodiments, a new IgG-like antibody can be prepared by using the method described above, with strong binding affinity to FcRn and prolonged serum half-life. Besides, it is to be noted that the binding affinity between the modified IgG-like antibody and its specific antigen is maintained with respect to the wild-type IgG-like antibody.

The type of antibody producing cell used is not particularly limited as long as the modified IgG-like antibody can be efficiently expressed and secreted, for example, the antibody producing cell can be cells derived from mouse, rat, rabbit, human being and the like. In embodiments, the antibody producing cell is a 293 cell. Thus, the modified IgG-like antibody can be produced easily, with a high yield and an excellent effect.

Further, the type of expression vector and the specific transfection method are not particularly limited as long as the modified IgG-like antibody can be expressed.

In addition, it should be noted that the term "amino acid" as used herein means any one of 20 natural amino acids or non-natural analogs thereof which may be present at a specific and defined position. The 20 natural amino acids can be abbreviated to a three-letter code or a one-letter code:

| | | |
|---|---|---|
| Alanine | ala | A |
| Arginine | arg | R |
| Asparagine | asn | N |
| Aspartic acid | asp | D |
| Asparagine or Aspartic acid | asx | B |
| Cysteine | cys | C |
| Glutamic acid | glu | E |
| Glutamine | gln | Q |
| Glutamine or Glutamic acid | glx | Z |
| Glycine | gly | G |
| Histidine | his | H |
| Isoleucine | ile | I |
| Leucine | leu | L |
| Lysine | lys | K |
| Methionine | met | M |
| Phenylalanine | phe | F |
| Proline | pro | P |
| Serine | ser | S |
| Threonine | thr | T |
| Tryptophan | try | W |
| Tyrosine | tyr | Y |
| Valine | val | V |

The expression "amino acid position n", such as amino acid positions 254, 308 and 434, refers to the specific amino acid position in the amino acid sequence of a protein. For the Fc fragment of the present disclosure, the amino acid position can be numbered according to the EU index in Kabat.

The additional aspects and advantages of the present disclosure will be set forth partly in the following description, part of which will become apparent from the description or understood from the practice of the present disclosure.

### BRIEF DESCRIPTION OF THE DRAWINGS

The above and/or additional aspects and advantages of the present disclosure will become apparent and readily understood from the description of examples in combination with the following figures, in which:
Figure 1 is a graph showing ELISA results of H8L2 and H2L2 antibodies binding to PD-1 according to some embodiments of the present disclosure;
Figure 2 is a graph showing competitive ELISA results of H8L2 and H2L2 antibodies competing with PdL1 on binding Pd-1 according to some embodiments of the present disclosure;
Figure 3 is a graph showing competitive ELISA results of H8L2 and H2L2 antibodies competing with PdL2 on binding Pd-1 according to some embodiments of the present disclosure;
Figure 4 is a graph showing the Kinetic characteristic parameters of H8L2 and H2L2 antibodies according to some embodiments of the present disclosure;
Figure 5 is a graph showing content of IL-2 secreted by T cells under stimulation of H8L2 and H2L2 antibodies via blocking the activation of PD-1 protein according to some embodiments of the present disclosure;
Figure 6 is a graph showing content of IFN gamma secreted by T cells under stimulation of H8L2 and H2L2 antibodies via blocking the activation of PD-1 protein according to some embodiments of the present disclosure;
Figure 7 is a graph showing concentration-time curves of H8L2 and H2L2 antibodies measured by ELISA in a serum concentration study of Cynomolgus monkey (*Macaca fascicularis*) according to some embodiments of the present disclosure;
Figure 8 is a graph showing individual plasma concentration after administration of H8L2 antibody in 1 mg/kg in a pharmacokinetic study of Cynomolgus monkey (*Macaca fascicularis*) according to some embodiments of the present disclosure;
Figure 9 is a graph showing individual plasma concentration after administration of H8L2 antibody in 3 mg/kg in a pharmacokinetic study of Cynomolgus monkey (*Macaca fascicularis*) according to some embodiments of the present disclosure;
Figure 10 is a graph showing individual plasma concentration after administration of H8L2 antibody in 10 mg/kg in a pharmacokinetic study of Cynomolgus monkey (*Macaca fascicularis*) according to some embodiments of the present disclosure;
Figure 11 is a graph showing individual plasma concentration after administration of wild-type H2L2 antibody in 10 mg/kg in a pharmacokinetic study of Cynomolgus monkey (*Macaca fascicularis*) according to some embodiments of the present disclosure;
Figure 12 is a graph showing effective average half-life of wild-type H2L2 and H8L2 antibodies in a pharmacokinetic study of Cynomolgus monkey (*Macaca fascicularis*) according to some embodiments of the present disclosure.

### DETAILED DESCRIPTION

Reference will be made in detail to examples of the present disclosure. It would be appreciated by those skilled in the art that the following examples are explanatory, and cannot be construed to limit the scope of the present disclosure. If the specific technology or conditions are not specified in the examples, a step will be performed in accordance with the techniques or conditions described in the literature in the art (for example, referring to J. Sambrook, et al. (translated by Huang PT), Molecular Cloning: A Laboratory Manual, 3rd Ed., Science Press) or in accordance with the product instructions. If the manufacturers of reagents or instruments are not specified, the reagents or instruments may be commercially available, for example, from Illumina Company.

### Example 1 Protein expression of H8L2 antibody

For humanized H2L2 antibody (IgG-like antibody against PD-1), amino acids at positions 254, 308, and 434 in the FcRn-binding site of the heavy chain constant region were respectively mutated to threonine, proline and alanine, giving in a variant named as IgG-like antibody H8L2 against PD-1 (i.e. H8L2 antibody).

That is, the H8L2 antibody of interest has a threonine mutation at amino acid position 254, a proline mutation at amino acid position 308 and an alanine mutation at amino acid position 434 in the FcRn-binding site of the heavy chain constant region, with remaining amino acids unchanged, compared to the humanized H2L2 antibody.

In practice, the nucleic acid sequence encoding the humanized H8L2 antibody which is formed via Gene Synthesis was constructed into an expression vector, which was transfected into a mammalian cell 293 cell. After transfection, the humanized H8L2 antibody was expressed and secreted by the mammalian cell 293 cell. Subsequently, such the humanized H8L2 antibody obtained was purified with a protein-A affinity column, thus obtaining purified humanized H8L2 antibody.

As described above, the difference between the humanized H2L2 and H8L2 antibodies only lies in the amino acids at positions 254, 308 and 434 in the FcRn-binding site of the heavy chain constant region, therefore just providing the amino acid sequence of the H8L2 antibody for reference.

Heavy chain of the humanized H8L2 antibody is of an amino acid sequence: 
EVQLVQSGGGLVQPGGSLKLSCAASGFTFSSYGMSWVRQAPGKGLDWVATISGGGR
DTYYPDSVKGRFTISRDNSKNNLYLQMNSLRAEDTALYYCARQKGEAWFAYWGQGTLVT
VSSASTKGPSVFPLAPCSRSTSESTAALGCLVKDYFPEPVTVSWNSGALTSGVHTFPAVLQS
SGLYSLSSVVTVPSSSLGTKTYTCNVDHKPSNTKVDKRVESKYGPPCPPCPAPEFLGGPSV
FLFPPKPKDTLMITRTPEVTCVVVDVSQEDPEVQFNWYVDGVEVHNAKTKPREEQFNST
YRVVSVLTPLHQDWLNGKEYKCKVSNKGLPSSIEKTISKAKGQPREPQVYTLPPSQEEMT
KNQVSLTCLVKGFYPSDIAVEWESNGQPENNYKTTPPVLDSDGSFFLYSRLTVDKSRWQE
GNVFSCSVMHEALHAHYTQKSLSLSLGK (SEQ ID NO: 1),
 in which the heavy chain variable region of the H8L2 antibody is underlined; and the mutation sites of the H8L2 antibody (relative to H2L2 antibody) are boxed, respectively being amino acid mutations at positions 254, 308 and 434 in the FcRn-binding site of the heavy chain constant region.

Specifically, for the amino acid mutations in the FcRn-binding site of the heavy chain constant region of H8L2 antibody, the amino acid at position 254 is mutated into threonine from serine, the amino acid at position 308 is mutated into proline from valine, and the amino acid at position 254 is mutated into alanine from asparagine, compared to the humanized H2L2 antibody.

Nucleic acid sequence encoding the heavy chain of the humanized H8L2 antibody is below:
ATGGGATGGAGCTGTATCATCCTCTTCTTGGTAGCAACAGCTACAGGCGCGCACTCCGAGGTGCAGCTGGTGCAGTCTGGCGGCGGACTGGTGCAGCCCGGCGGGTCACTGAAGCTGAGCTGCGCCGCTTCCGGCTTCACCTTTAGCTCCTACGGAATGTCCTGGGTGCGACAGGCACCCGGGAAGGGGCTGGACTGGGTCGCTACTATCTCAGGAGGCGGGAGAGACACCTACTATCCTGATAGCGTCAAGGGCCGGTTCACAATTAGCCGGGACAACAGCAAGAACAATCTGTACCTGCAGATGAACAGCCTGAGGGCTGAGGATACTGCACTGTACTATTGTGCCCGCCAGAAGGGCGAAGCATGGTTTGCCTATTGGGGCCAGGGAACCCTGGTCACCGTCTCCTCAGCTTCCACCAAGGGCCCATCCGTCTTCCCCCTGGCGCCCTGCTCCAGGAGCACCTCCGAGAGCACAGCCGCCCTGGGCTGCCTGGTCAAGGACTACTTCCCCGAACCGGTGACGGTGTCGTGGAACTCAGGCGCCCTGACCAGCGGCGTGCACACCTTCCCGGCTGTCCTACAGTCCTCAGGACTCTACTCCCTCAGCAGCGTGGTGACTGTGCCCTCCAGCAGCTTGGGCACGAAGACCTACACCTGCAACGTAGATCACAAGCCCAGCAACACCAAGGTGGACAAGAGAGTTGAGTCCAAATATGGTCCCCCATGCCCACCATGCCCAGCACCTGAGTTCCTGGGGGGACCATCAGTCTTCCTGTTCCCCCCAAAACCCAAGGACACTCTCATGATCACCCGGACCCCTGAGGTCACGTGCGTGGTGGTGGACGTGAGCCAGGAAGACCCCGAGGTCCAGTTCAACTGGTACGTGGATGGCGTGGAGGTGCATAATGCCAAGACAAAGCCGCGGGAGGAGCAGTTCAACAGCACGTACCGTGTGGTCAGCGTCCTCACCCCCCTGCACCAGGACTGGCTGAACGGCAAGGAGTACAAGTGCAAGGTCTCCAACAAAGGCCTCCCGTCCTCCATCGAGAAAACCATCTCCAAAGCCAAAGGGCAGCCCCGAGAGCCACAGGTGTACACCCTGCCCCCATCCCAGGAGGAGATGACCAAGAACCAGGTCAGCCTGACCTGCCTGGTCAAAGGCTTCTACCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAGGCTAACCGTGGACAAGAGCAGGTGGCAGGAGGGGAATGTCTTCTCATGCTCCGTGATGCATGAGGCTCTGCACGCCCACTACACACAGAAGAGCCTCTCCCTGTCTCTGGGTAAA (SEQ ID NO: 2), in which the nucleic acid sequence encoding the heavy chain variable region is underlined.

Light chain of the humanized H8L2 antibody is of an amino acid sequence:
DIVLTQSPASLAVSPGQRATITCRASESVDNYGISFMNWFQQKPGQPPKLLIYAASNKGTGVPARFSGSGSGTDFTLNINPMEENDTAMYFCQQSKEVPWTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 3), in which the light chain variable region of the H8L2 antibody is underlined.

The nucleic acid encoding the light chain of the humanized H8L2 antibody is of a nucleotide sequence:
ATGGGATGGAGCTGTATCATCCTCTTCTTGGTAGCAACAGCTACAGGCGTGCACTCCGATATTGTGCTGACTCAGAGCCCTGCTTCCCTGGCCGTGTCTCCAGGACAGCGAGCTACCATCACATGCAGAGCATCTGAGAGTGTGGACAACTACGGAATTAGTTTCATGAATTGGTTTCAGCAGAAGCCCGGCCAGCCCCCTAAACTGCTGATCTATGCCGCCAGCAACAAGGGCACCGGGGTGCCTGCTCGATTCTCAGGAAGCGGCTCCGGGACAGACTTTACTCTGAACATTAACCCAATGGAGGAAAATGATACAGCAATGTACTTCTGCCAGCAGAGCAAGGAGGTGCCCTGGACCTTTGGCGGGGGAACAAAGCTGGAAATCAAACGAACTGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCTTCAACAGGGGAGAGTGT (SEQ ID NO: 4), in which the nucleic acid sequence encoding the light chain variable region is underlined.

### Example 2 ELISA experiment of recombinant humanized H8L2 antibody

The humanized H2L2 antibody and the humanized H8L2 antibody prepared in Example 1 were subjected to an ELISA binding experiment and a competitive ELISA experiment for comparison, as described in detail below.

### 2.1 ELISA binding experiments of 18A10 H8L2 and 18A10 H2L2 antibodies

Specifically, the ELISA binding experiment was conducted as follows.

### Step a): Antigen coating

An ELISA plate was coated with PD-1-his antigen in a concentration of 0.25 µg/ml (100 µl per well) by incubation at 4°C overnight.

### Step b): Blocking

The ELISA plate coated with the PD-1-his antigen was blocked with 1% BSA in the PBS buffer at 37°C for 2 hours and washed with 1×PBST buffer containing 1% Tween-20 for three times, with gently patting to dryness.

### Step c): Incubation with primary antibody

The 18A10 H8L2 and 18A10 H2L2 antibodies were respectively diluted from 2µg/ml in series by 1:3, with 7 gradient antibody solutions obtained. The 7 gradient antibody solutions of each of the 18A10 H8L2 and 18A10 H2L2 antibodies were respectively added into the blocked ELISA plate for incubation at 37°C for 1 hour, with the PBS solution as a blank control.

### Step d): Incubation with secondary antibody

After the ELISA plate was washed with the PBST buffer for three times and gently patted to dryness, goat anti-human IgG-HRP (H+L) as a secondary antibody in 1:10000 dilution (100 µl per well) was added for incubation at 37°C for 1 hour.

### Step e): Developing

After the ELISA plate was washed with the PBST buffer for three times and gently patted to dryness again, 3,3',5,5'-Tetramethylbenzidine (TMB) as a developer in 100 µl per well was added for incubation at room temperature for 5 to 10 minutes.

### Step f): Termination of developing

2M H₂SO₄ solution in 50 µl per well was added to terminate developing.

### Step g): Reading

The absorbance of solution in each well was measured with the microplate reader at a wavelength of 450 nm.

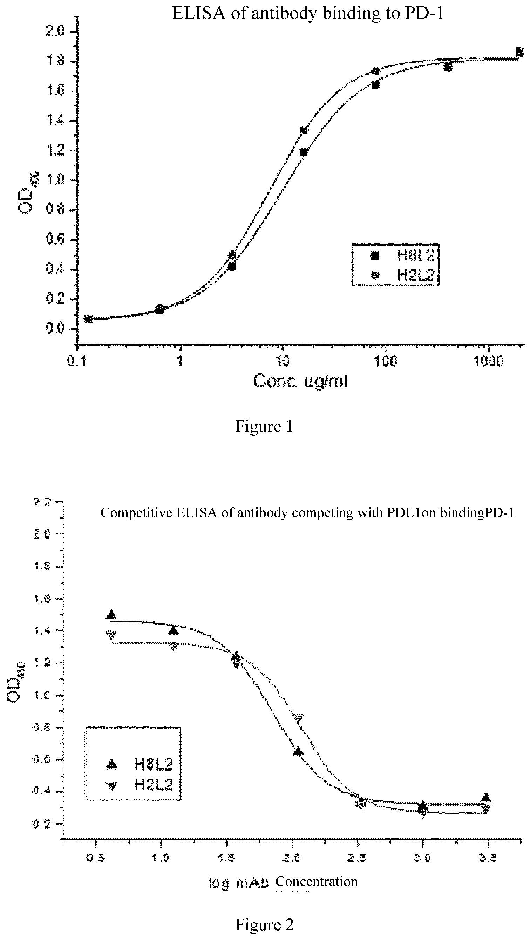

Figure 1 shows the results, from which the EC₅₀ values of H8L2 and H2L2 binding to PD-1 are respectively calculated to be 0.04 nM and 0.05 nM. As can be seen from Figure 1, the mutation in the FcRN-binding site has no effect on the binding of antibody to PD-1.

| Series dilution of antibody | 18A10 H8L2 | | 18A10 H2L2 | |
|---|---|---|---|---|
| 2 µg/ml | 1.881 | 1.84 | 1.9 | 1.847 |
| 1:3 | 1.756 | 1.756 | 1.784 | 1.757 |
| 1:9 | 1.661 | 1.628 | 1.716 | 1.736 |
| 1:27 | 1.214 | 1.156 | 1.341 | 1.34 |
| 1:81 | 0.429 | 0.419 | 0.514 | 0.491 |
| 1:243 | 0.127 | 0.125 | 0.146 | 0.14 |
| 1:729 | 0.072 | 0.066 | 0.068 | 0.069 |
| 0 | 0.052 | 0.05 | 0.054 | 0.048 |

### 2.2 Competitive ELISA experiments of 18A10 H8L2 and 18A10 H2L2 antibodies with PDL1

Specifically, the competitive ELISA experiment was conducted as follows.

### Step a): Antigen coating

A 96-well ELISA plate was coated with PD-1-hIgGFc antigen in a concentration of 0.5 µg/ml (50 µl per well) by incubation at 4°C overnight.

### Step b): Blocking

After washed with the PBST buffer for three times and gently patted to dryness, the 96-well ELISA plate was blocked with 1% BSA in the PBS buffer at 37°C for 2 hours and washed with the 1×PBST buffer containing 1% Tween-20 for three times.

### Step c): Incubation with primary antibody

The 18A10 H8L2 and 18A10 H2L2 antibodies were respectively diluted from 6µg/ml in series by 1:3, with 7 gradient antibody solutions obtained. The 7 gradient antibody solutions of each of the 18A10 H8L2 and 18A10 H2L2 antibodies (50µl per well) were respectively added into the blocked 96-well ELISA plate for incubation at room temperature for 10 minutes, with the PBS solution as a blank control.

### Step d): Incubation with ligand

0.6 µg/ml of PDL1-mIgG2aFc solution in 50 µl per well was added for incubation at 37°C for 1 hour.

### Step e): Incubation with secondary antibody

After the 96-well ELISA plate was washed with the PBST buffer for three times and gently patted to dryness, goat anti-mouse IgG-HRP (H+L) as a secondary antibody in 1:5000 dilution (50 µl per well) was added for incubation at 37°C for 1 hour.

### Step f): Developing

After the 96-well ELISA plate was washed with the PBST buffer for three times and gently patted to dryness again, TMB as a developer in 50 µl per well was added for incubation at room temperature for 5 to 10 minutes.

### Step g): Termination of developing

2M H₂SO₄ solution in 50 µl per well was added to terminate developing.

### Step h): Reading

The absorbance of solution in each well was measured with the microplate reader at a wavelength of 450 nm.

Figure 2 shows the results, from which the EC₅₀ values of H8L2 and H2L2 antibodies competitively binding to PD-1 in the presence of PD-L1 are respectively 0.474 nM and 0.783 nM, which demonstrates the mutation in the FcRN-binding site has no effect on the competitively binding to PD-1 in the presence of PD-L1.

| Series dilution of antibody | 18A10 H8L2 | | 18A10 H2L2 | |
|---|---|---|---|---|
| 3 µg/ml | 0.367 | 0.348 | 0.301 | 0.294 |
| 1:3 | 0.329 | 0.293 | 0.26 | 0.276 |
| 1:9 | 0.325 | 0.34 | 0.335 | 0.31 |
| 1:27 | 0.658 | 0.642 | 0.828 | 0.883 |
| 1:81 | 1.275 | 1.194 | 1.191 | 1.214 |
| 1:243 | 1.454 | 1.344 | 1.276 | 1.336 |
| 1:729 | 1.489 | 1.5 | 1.385 | 1.369 |
| 0 | 2.113 | 2.067 | 2.09 | 1.417 |
| Ligand | PDL1-mIgG2aFc 0.3 µg/ml | | | |

### 2.3 Competitive ELISA experiments of 18A10 H8L2 and 18A10 H2L2 antibodies with PDL2

Specifically, the competitive ELISA experiment was conducted as follows.

### Step a): Antigen coating

A 96-well ELISA plate was coated with PD-1-hIgGFc antigen in a concentration of 1.0 µg/ml (100 µl per well) by incubation at 4°C overnight.

### Step b): Blocking

After washed with the PBST buffer for three times and gently patted to dryness, the 96-well ELISA plate was blocked with 1% BSA in the PBS buffer at 37°C for 2 hours and washed with the 1×PBST buffer containing 1% Tween-20 for four times.

### Step c): Incubation with primary antibody

The 18A10 H8L2 and 18A10 H2L2 antibodies were respectively diluted from 20µg/ml in series by 1:3, with 7 gradient antibody solutions obtained. The 7 gradient antibody solutions of each of the 18A10 H8L2 and 18A10 H2L2 antibodies (50µl per well) were respectively added into the blocked 96-well ELISA plate for incubation at room temperature for 10 minutes, with the PBS solution as a blank control.

### Step d): Incubation with ligand

1.0 µg/ml of PDL2-his tag solution in 50 µl per well was added for incubation at 37°C for 1 hour.

### Step e): Incubation with secondary antibody

After the 96-well ELISA plate was washed with the PBST buffer for five times and gently patted to dryness, HRP-conjugated monoclonal mouse anti-his tag as a secondary antibody in 1:750 dilution (50 µl per well) was added for incubation at 37°C for 1 hour.

### Step f): Developing

After the 96-well ELISA plate was washed with the PBST buffer for six times and gently patted to dryness again, TMB as a developer in 100 µl per well was added for incubation at room temperature for 30 minutes.

### Step g): Termination of developing

2M H₂SO₄ solution in 50 µl per well was added to terminate developing.

### Step h): Reading

The absorbance of solution in each well was measured with the microplate reader at a wavelength of 450 nm.

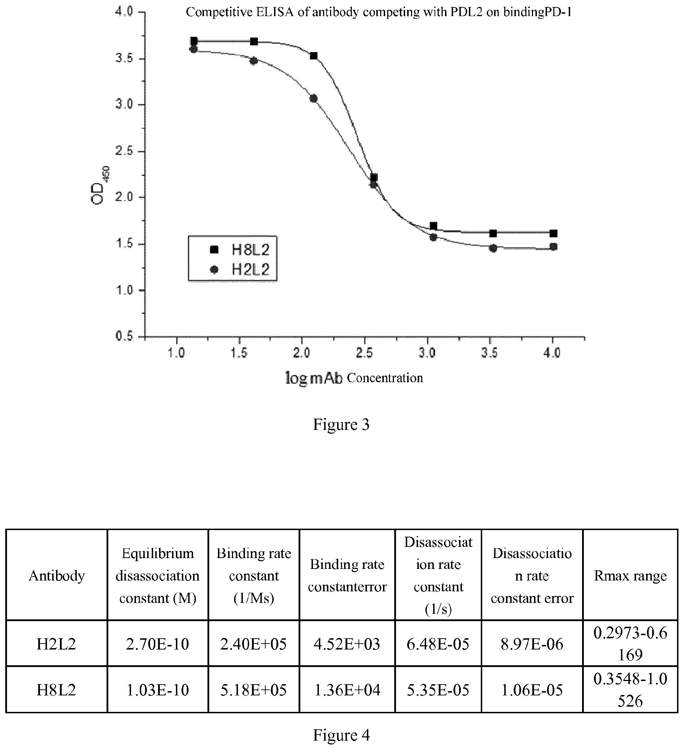

Figure 3 shows the results, from which the EC₅₀ values of H8L2 and H2L2 antibodies competitively binding to PD-1 in the presence of PDL2 are respectively 1.83 nM and 1.58 nM, which demonstrates the mutation in the FcRN-binding site has no effect on the competitively binding to PD-1 in the presence of PDL2.

| Series dilution of antibody | 18A10 H8L2 | | 18A10 H2L2 | |
|---|---|---|---|---|
| 10µg/ml | 1.681 | 1.551 | 1.493 | 1.454 |
| 1:3 | 1.628 | 1.596 | 1.46 | 1.455 |
| 1:9 | 1.74 | 1.643 | 1.585 | 1.566 |
| 1:27 | 2.101 | 2.331 | 2.206 | 2.072 |
| 1:81 | 3.485 | 3.577 | 3.139 | 3 |
| 1:243 | 3.682 | 3.685 | 3.476 | 3.475 |
| 1:729 | 3.692 | 3.682 | 3.773 | 3.432 |
| Blank | 0.401 | 0.28 | | |
| Ligand | PDL2-his tag 0.5µg/ml | | | |

### Example 3 Determination of kinetic characteristic parameters of H8L2 and H2L2 antibodies with Fortebio molecular interaction instrument

The kinetic characteristic parameters of H8L2 antibody prepared in Example 1 and H2L2 antibody were determined using the Fortebio molecular interaction instrument for comparison, which are described in detail below.

The biotin-labeled PD-1 antigen was immobilized on the surface of the SA sensor. After equilibration with the PBST buffer, the H8L2 antibody, diluted in series by 1:3 with PBST (200 nM, 66.67 nM, 22.22 nM, 7.41 nM, 2.47 nM, 0.82 nM, 0.27 nM and 0 nM respectively), was applied to the SA sensor for binding to the biotin-labeled PD-1 antigen, after which PBST was applied to the SA sensor for disassociation. Assay for H2L2 antibody is the same as H8L2 antibody. Results of kinetic characteristic parameters of the H8L2 and H2L2 antibodies are shown in Figure 4, from which it can be seen the mutation in the FcRN-binding site has no effect on the kinetic characteristic parameters of antibody.

### Example 4 Assay of biological activity of antibody against PD1 under mixed lymphatic reaction

T lymphocytes were assayed for IL-2 and IFN gamma secretion under stimulation of H8L2 antibody prepared in Example 1 and H2L2 antibody by the mixed lymphocyte reaction (MLR) for comparison, which is described in detail below.

For MLR, T cells (TC) and dendritic cells (DC) from different human sources were mixed, such that the T cells secrete IL-2 and IFN gamma under antigen presenting function of the DC cells. Specifically, monocytes in the blood differentiate into immature DC cells under the induction of cytokines GM-CSF and IL-4, after which the immature DC cells were induced to maturation via stimulation of tumor necrosis factor alpha (TNFα). Subsequently, the matured DC cells and allogeneic TC cells were mixed and cultured for 5 days, thereafter the secreted IL-2 and IFN gamma in cell supernatant were determined. In this example, the TC cells (1×10⁵ per well) and the matured DC cells (1×10⁴ per well) were mixed in a 96 well plate, and then cultured in the presence of individual antibodies in eight gradient concentrations (i.e. from 10 µM to 0.09765625 nM) for 5 days, after which the amount of IL-2 in cell supernatant was detected with an IL-2 assay kit. Similarly, the TC cells (1×10⁵ per well) and the matured DC cells (1×10⁴ per well) were mixed in a 96 well plate, and then cultured in the presence of individual antibodies in five gradient concentrations (i.e. from 300 nM to 0.1 nM) for 5 days, after which the amount of IFN gamma in the cell supernatant was detected with an IFN gamma assay kit.

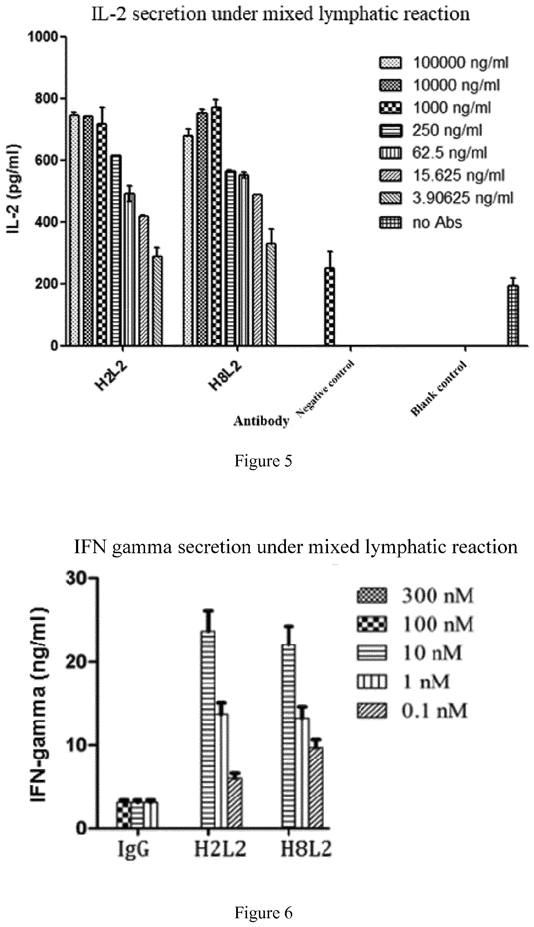

Figure 5 shows the content of IL-2 secreted by T cells under the stimulation of the H8L2 and H2L2 antibodies respectively, from which it can be seen that the H8L2 and H2L2 antibodies are capable of stimulating T cells to secrete IL-2 in an effective manner, which demonstrates that the mutation in the FcRN-binding site has no effect on the IL-2 secretion by T cells under the stimulation of antibody.

Figure 6 shows the content of IFN gamma secreted by T cells under the stimulation of the H8L2 and H2L2 antibodies respectively, from which it can be seen that the H8L2 and H2L2 antibodies are capable of stimulating T cells to secrete IFN gamma in an effective manner, which demonstrates that the mutation in the FcRN-binding site has no effect on the IFN gamma secretion by T cells under the stimulation of antibody. The "IgG" in Figure 6 is an isotype antibody as a control.

### Example 5 Serum concentration study of Cynomolgus monkey (Macaca fascicularis)

Serum concentrations of H8L2 antibody prepared in Example 1 and H2L2 antibody in Cynomolgus monkey (*Macaca fascicularis*) were respectively detected for comparison, which is described in detail below.

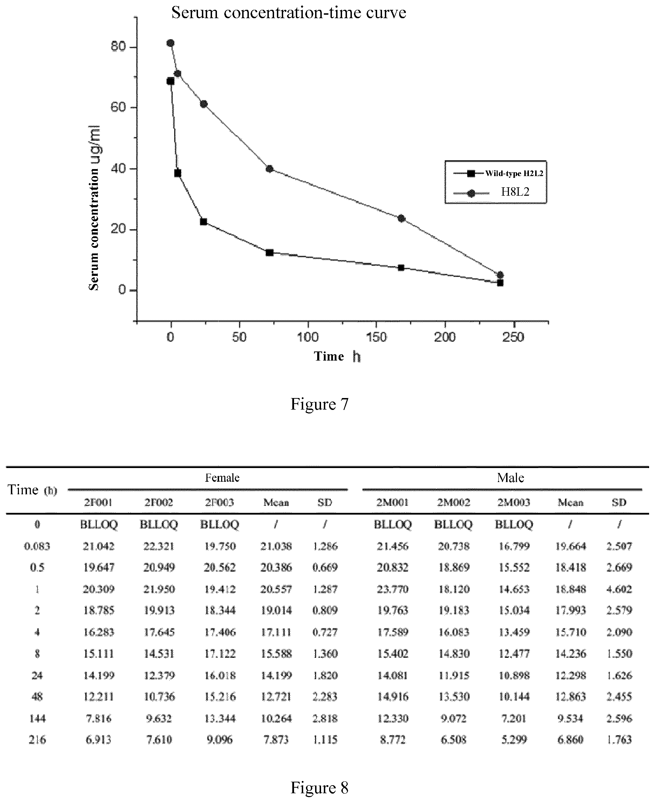

Four Cynomolgus monkeys (*Macaca fascicularis*) were randomly divided into 2 groups as their body weights, respectively named as H8L2 group and H2L2 group, with 2 animals per group. Each group was administered with its individual antibody in a dosage of 1 mg/kg by intravenous injection, with whole blood sampled before administration and after administration 5 minutes, 5 hours, 24 hours, 72 hours, 168 hours and 240 hours respectively. Blood serum was separated from the whole blood and the contents of H8L2 and H2L2 antibodies were respectively measured by the ELISA method, which can be seen in Figure 7 and the table below.

| | Serum concentration (ug/ml) | | | | | |
|---|---|---|---|---|---|---|
| Time (h) | H2L2 | H2L2 | Mean | H8L2 | H8L2 | Mean |
| 0 | 0 | 0 | 0 | 0 | 0 | 0 |
| 0.015 (Tmax) | 62.5 | 75 | 68.75 | 95 | 67.5 | 81.25 |
| 5 | 32.5 | 45 | 38.75 | 90 | 52.5 | 71.25 |
| 24 | 22.5 | 22.5 | 22.5 | 80 | 42.5 | 61.25 |
| 72 | 15 | 10 | 12.5 | 50 | 30 | 40 |
| 168 | 10 | 5 | 7.5 | 27.5 | 20 | 23.75 |
| 240 | 5 | 0 | 2.5 | 0 | 10 | 5 |

### Example 6 Pharmacokinetic Study of Cynomolgus monkey (Macaca fascicularis)

Pharmacokinetics of H8L2 antibody prepared in Example 1 and H2L2 antibody in Cynomolgus monkey (*Macaca fascicularis*) was studied for comparison, which is described in detail below.

24 Cynomolgus monkeys (*Macaca fascicularis*) were randomly divided into 4 groups as their body weights, respectively named as a H2L2 group (10 mg/kg) and three H8L2 groups in different dosages (that is low: 1 mg/kg, medium: 3 mg/kg and high: 10 mg/kg), with 6 animals per group (male and female half for each group). Each group was administered with its individual antibody by intravenous injection, with whole blood sampled before administration and after administration 5 minutes, 30 minutes, 1 hour, 2 hours, 4 hours, 8 hours, 24 hours, 48 hours, 144 hours and 216 hours respectively. Blood serum was separated from the whole blood and the contents of H8L2 and H2L2 antibodies were respectively measured by the ELISA method, with relevant pharmacokinetic parameters calculated by PhoenixWinNonlin (Pharsight) 6.4.

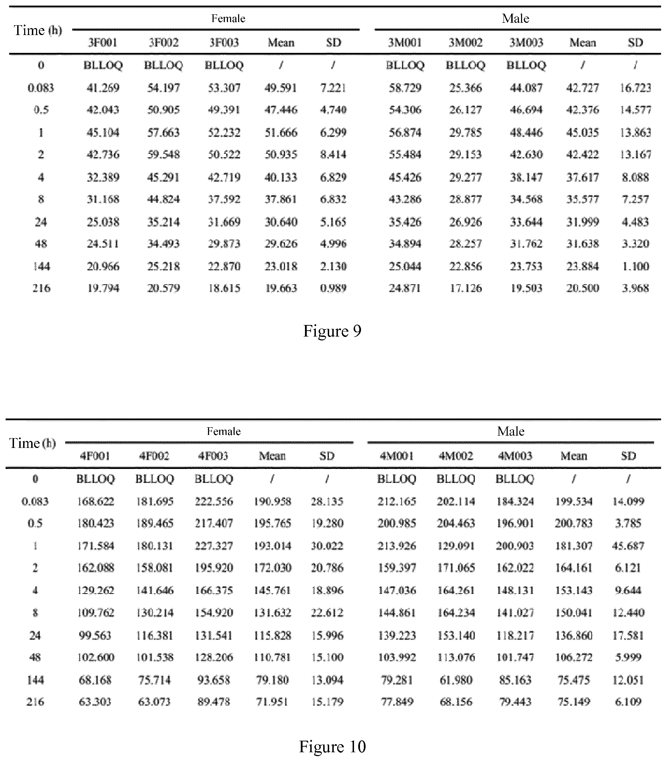

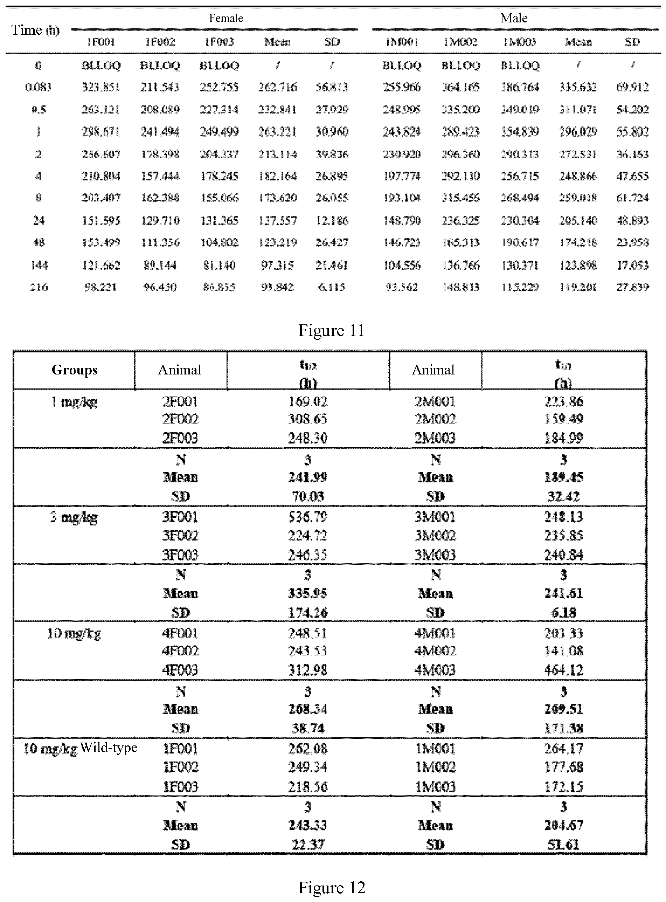

Before single administration, the serum concentration of H2L2 and H8L2 antibodies in all cynomolgus monkey individuals is below the lower limit of quantitation. After the administration, the serum concentration of H8L2 antibody in the cynomolgus monkeys of the three H8L2 groups increases as the administration dosage, in which the effective average half-life of the low dosage group (i.e. 1 mg/kg), the medium dosage group (i.e. 3 mg/kg) and the high dosage group (i.e. 10 mg/kg) is respectively 215.72 hours (refer to Figure 8), 288.78 hours (refer to Figure 9) and 268.92 hours (refer to Figure 10). Further, the effective average half-life of the wild-type H2L2 group (in a dosage of 10 mg/kg) is 224 hours (refer to Figure 11). It can be seen, the H8L2 group exhibits longer effective average half-life than the wild-type H2L2 group under a same dosage of 10 mg/kg (refer to Figure 12).

### Example 7 Anti-tumor effect of H8L2 antibody in subcutaneously implanted tumor MiXeno model of human non-small cell lung cancer HCC827 cell line

Anti-tumor effect of H8L2 antibody prepared in Example 1 was investigated by using a subcutaneously implanted tumor MiXeno model established with human non-small cell lung cancer HCC827 cell line in NSG mice, which is described in detail below.

NSG mice, featured by non-obese diabetes (NOD), Prkdc_{scid} and IL2rgₙᵤₗₗ deletion or mutation, have highest immune deficiency and thus become a most suitable tool for human-derived cell transplantation, without rejection to human-derived cells and tissues. Based on the above, the present inventors evaluated pharmaceutical effect of H8L2 antibody *in vivo* by means of a graft-versus-host disease (GVHD) model established by adoptive transplantation of human peripheral blood mononuclear cells (PBMC) into the NSG mouse. Besides, the present inventors have established the subcutaneously implanted tumor model (i.e. MiXeno model) by using the NSG mouse, and further discovered anti-tumor effect of H8L2 antibody on the subcutaneously implanted tumor MiXeno model of human non-small cell lung cancer HCC827 cell line.

Specifically, HCC827 cells were inoculated into the right side of the back of each 40 NCG mouse (32 experimental mice plus 8 mice for spare) in a dosage of 5×10⁶ cells per mouse by subcutaneous injection on day 0 (Day 0). On day 6 post inoculation (Day 6), 32 NCG mice with a tumor size up to 66 mm³ were divided into 4 groups with 8 mice per group, and each mouse was subjected to tail-intravenous transplantation of 0.1 ml PBMC (suspended in the PBS buffer). For the four groups (i.e. 32 mice), H8L2 5 mg/kg treatment group (Group 1), H8L2 10 mg/kg treatment group (Group 2), Opdivo 5 mg/kg treatment group (Group 3) as a positive control, and isotype antibody (Human IgG4) 5mg/kg group (Group 4) as a control were administrated intravenously via the tail vein at day 6, day 9, day 13, day16, day 19 and day 22 post inoculation respectively, with a total of 6 administrations as shown in Table 1. The efficacy was evaluated according to the relative tumor growth inhibition value (TGI_{RTV}), and the safety was evaluated according to the body weight change and death of mice.

**Table 1 Experimental design for anti-tumor effect of H8L2 antibody on the MiXeno model of human non-small cell lung cancer HCC827 cell line**

| **Groups** | **n** | **(Day 0) Subcutaneous implantation** | **PBMC** | **Treatment** | **Dosage concentration (mg/kg)** | **Administration mode** | **Dosage volume** | **Administration time** |
|---|---|---|---|---|---|---|---|---|
| 1 | 8 | 5 × 10⁶/100 µL | tumor size of 66mm³; intravenous injection of 0.1 ml PBMC suspension per mouse on day 6 post inoculation of tumor cell | Anti-Hel-hIg G4 | 5 | intravenous (i.v.) | 10µl/g | on day 6, day 9, day 13, day16, day 19 and day 22 post inoculation respectively |
| 2 | 8 | | | Opdivo | 5 | intravenous (i.v.) | 10µl/g | |
| 3 | 8 | | | H8L2 | 5 | intravenous (i.v.) | 10µl/g | |
| 4 | 8 | | | H8L2 | 10 | intravenous (i.v.) | 10µl/g | |

| | | | | | | | | |
|---|---|---|---|---|---|---|---|---|
| Note: Dosage volume is 10 µl/g; n represents the number of mice; Day 0 represents the day when tumor cells are inoculated; i.v. represents intravenous administration via the tail vein | | | | | | | | |

With respect to the isotype antibody (Human IgG4) 5mg/kg group as a control, the H8L2 10 mg/kg treatment group exhibits a significant inhibition of tumor growth on day 9 and 13 post inoculation of tumor cell, with the relative tumor growth inhibition value (TGI_{RTV}) of 30% (p=0.007) and 30% (p=0.039) respectively; the H8L2 5 mg/kg treatment group also shows a significant inhibition of tumor growth on day 9 and 13 post inoculation of tumor cell, with the relative tumor growth inhibition value (TGI_{RTV}) of 18% (p=0.049) and 25% (p=0.041) respectively; while the Opdivo 5 mg/kg treatment group does not display a more significant inhibition of tumor growth on day 9 and 13 post inoculation of tumor cell, with the relative tumor growth inhibition value (TGI_{RTV}) of 17% (p=0.084) and 23% (p=0.073) respectively, refer to Table 2. The results demonstrate that the H8L2 antibody is capable of significantly inhibiting the tumor growth of the tumor MiXeno model of human non-small cell lung cancer HCC827 cell line, with even better efficacy than the Opdivo group which is used as a positive control. Further, the H8L2 5 mg/kg treatment group and the H8L2 10 mg/kg treatment group do not develop drug-related toxicity (such as severe weight loss or death) similar with the Opdivo 5 mg/kg treatment group within 16 days from the first administration (i.e. Day 6 to Day 22 post inoculation), indicating well tolerance for the treatment of H8L2 antibody.

**Table 2 Pharmaceutical effect assay on the tumor MiXeno model of human non-small cell lung cancer HCC827 cell line**

| **Treatment groups** | **Day** | **Tumor size (mm³) (Mean ± SEM)** | **Relative tumor size (Mean ± SEM)** | **TGI_{RTV} (%)** | **P value¹** |
|---|---|---|---|---|---|
| G1 Human lgG4 5 mg/kg | 9 | 88±8 | 1.33±0.08 | - | - |
| | 13 | 116±12 | 1.76±0.15 | - | - |
| | 16 | 132±15 | 2.00±0.17 | - | - |
| G2 Opdivo 5 mg/kg | 9 | 74±8 | 1.11±0.09 | 17 | 0.084 |
| | 13 | 91±14 | 1.35±0.15 | 23 | 0.073 |
| | 16 | 109±11 | 1.64±0.12 | 18 | 0.106 |
| G3 H8L2 5mg/kg | 9 | 72±6 | 1.09±0.07 | 18 | 0.049 |
| | 13 | 87±10 | 1.32±0.12 | 25 | 0.041 |
| | 16 | 103±12 | 1.57±0.17 | 21 | 0.097 |
| G4 H8L2 10mg/kg | 9 | 62±8 | 0.93±0.09 | 30 | 0.007 |
| | 13 | 82±14 | 1.22±0.18 | 30 | 0.039 |
| | 16 | 110±24 | 1.63±0.33 | 18 | 0.340 |

| | | | | | |
|---|---|---|---|---|---|
| Note: **P value¹** is obtained by comparing with the isotype antibody (Human IgG4) 5mg/kg group | | | | | |

The H8L2 antibody (i.e. monoclonal antibody against PD-1) shows significant inhibition of tumor growth on the tumor MiXeno model of human non-small cell lung cancer HCC827 cell line when injected at the administration dosage of 10 mg/kg and 5 mg/kg respectively, where the H8L2 antibody at the administration dosage of 10mg/kg exhibits even more significant inhibition of tumor growth and displays better efficacy over the Opdivo 5 mg/kg treatment group as the positive control, with well tolerance for the tumor-bearing mice under the dosage of both 10mg/kg and 5mg/kg.

### Example 8 Anti-tumor effect of H8L2 antibody in HuGEMM model of MC38 murine colorectal cancer cell line

The efficacy of H8L2 antibody prepared in Example 1 for treatment of colorectal cancer was pre-clinically validated in the PD-1 HuGEMM MC38-bearing mouse, which is described in detail below.

MC38 cell line is a murine colorectal cancer cell line derived from C57BL/6 mouse. The PD-1 HuGEMM model is a modeled mouse which is genetically engineered by replacing some fragments of murine PD-1 protein that interacts with PD-L1 protein molecule in the C57BL/6 mouse with corresponding human-derived protein.

MC38 cells were inoculated into the right side of each subject mice in a dosage of 1×10⁶ cells per mouse by subcutaneous injection. The mice with a tumor size up to 134 mm³ were randomly divided into 4 groups as the tumor size, with 8 mice per group and 4 mice per cage, named as Group 1 to Group 4, that is H8L2 5 mg/kg treatment group, H8L2 10 mg/kg treatment group, Keytruda 10 mg/kg treatment group as a positive control, and isotype antibody (Human IgG4) 5mg/kg group as a control respectively. The corresponding antibody for each group was administrated intravenously via the tail vein of mice, with a total of 6 administrations, refer to Table 3.

**Table 3 Experimental design for pharmaceutical effect assay**

| **Groups** | **Number** | **Treatment** | **Dosage (mg/kg)** | **Administration mode** | **Dosage regimen** |
|---|---|---|---|---|---|
| 1 | 8 | Isotype control | 10 | i.p. | BIW×3 |
| 2 | 8 | Keytruda | 10 | i.p. | BIW×3 |
| 3 | 8 | H8L2 | 5 | i.p. | BIW×3 |
| 4 | 8 | H8L2 | 10 | i.p. | BIW×3 |

On day 13 post grouping, the mice in the Group 1 have the average tumor size up to 1933.67 mm³, and the Group 2 (Keytruda 10 mg/kg treatment group, in a high dosage), the Group 3 (H8L2 5 mg/kg treatment group, in a low dosage) and the Group 4 (H8L2 10 mg/kg treatment group, in a high dosage) each have a tumor growth inhibition (TGI) (%) of 85%, 93% and 90% respectively, refer to Table 4; and the four groups respectively have a percentage weight change of 8.72%, 0.94%, -2.07% and 1.68%. Each of mice has no significant unexpected weight loss or death. The Groups 2 to 4 show statistically significant difference in inhibition effect compared to the group 1, each with P<0.05.

**Table 4 Anti-tumor effect of H8L2 antibody in PD-1 HuGEMM MC38-bearing mice**

| **Groups** | **Treatment** | **Tumor size on Day 0^{a} (mm³)** | **Tumor size on Day 13^{a} (mm³)** | **TGI (%)** | **T-C (day)** | ***P* value^{b}** |
|---|---|---|---|---|---|---|
| 1 | Isotype control 10 mg/kg | 132.86±14.78 | 1933.67±454.6 | - | - | - |
| 2 | Keytruda 10 mg/kg | 135.35±20.19 | 275.71±160.18 | 85 | 10 | <0.05 |
| 3 | H8L2 5 mg/kg | 133.70±17.67 | 133.72±80.59 | 93 | 14 | <0.05 |
| 4 | H8L2 10 mg/kg | 134.16±14.89 | 198.59±122.12 | 90 | >14 | <0.05 |

| | | | | | | |
|---|---|---|---|---|---|---|
| Note: a. data is represented in "mean ± standard error"; b. the significant difference among groups for tumor size is analyzed by using One-way ANOVA, where Groups 2 to 4 show a statistically significant difference in tumor size compared to Group 1, with P<0.05. | | | | | | |

For the Groups 2 to 4, the T-C values (when the tumor size of mouse reached up to 1000 mm³) were 10 days, 14 days and above 14 days respectively. Further, for the Groups 2 to 4, there were respectively 3 mice, 5 mice and 5 mice left in which the tumor has been regressed completely even for more than one month when the experiment was completed on day 55, refer to Table 5.

**Table 5 Raw data for tumor size of MC38 cell**

| | | Study day(s) | | | | | | | | | | | | | | | | |
|---|---|---|---|---|---|---|---|---|---|---|---|---|---|---|---|---|---|---|
| groups | ID | 0 | 3 | 6 | 10 | 13 | 17 | 20 | 24 | 27 | 31 | 34 | 38 | 41 | 45 | 48 | 52 | 55 |
| 01 | 11240 | 91.50 | 215.37 | 266.98 | 613.57 | 1171.37 | 1697.85 | 2643.02 | 4731.76 | 7309.75 | | | | | | | | |
| 01 | 11243 | 133.90 | 232.18 | 359.57 | 1304.79 | 3568.60 | | | | | | | | | | | | |
| 01 | 11250 | 80.84 | 109.37 | 143.14 | 271.79 | 466.01 | 894.52 | 1343.39 | 3027.15 | 4135.77 | | | | | | | | |
| 01 | 11260 | 119.44 | 189.27 | 546.36 | 1410.43 | 2834.20 | 5938.21 | | | | | | | | | | | |
| 01 | 11275 | 190.55 | 501.32 | 1112.25 | 1864.70 | 3346.82 | | | | | | | | | | | | |
| 01 | 11282 | 188.05 | 370.78 | 715.45 | 1249.03 | 2433.64 | 4743.60 | | | | | | | | | | | |
| 01 | 11285 | 154.20 | 312.96 | 497.55 | 884.87 | 1392.7 1 | 2469.10 | 3736.92 | | | | | | | | | | |
| 01 | 11286 | 104.36 | 174.07 | 177.68 | 201.64 | 256.00 | 276.76 | 419.35 | 799.84 | 1211.13 | 1774.66 | 2951.53 | 4747.39 | | | | | |
| 02 | 11248 | 125.08 | 136.50 | 118.99 | 40.80 | 17.53 | 19.47 | 39.80 | 61.94 | 111.33 | 248.10 | 525.66 | 1075.55 | 1492.19 | 2249.90 | 3687.06 | | |
| 02 | 11252 | 153.95 | 209.80 | 191.10 | 41.33 | 44.65 | 0.00 | 0.00 | 0.00 | 0.00 | 0.00 | 0.00 | 0.00 | 0.00 | 0.00 | 0.00 | 0.00 | 0.00 |
| 02 | 11257 | 116.34 | 235.53 | 425.25 | 668.02 | 1331.44 | 2715.30 | 5742.74 | | | | | | | | | | |
| 02 | 11259 | 257.74 | 414.24 | 200.04 | 59.12 | 0.00 | 0.00 | 0.00 | 0.00 | 0.00 | 0.00 | 0.00 | 0.00 | 0.00 | 0.00 | 0.00 | 0.00 | 0.00 |
| 02 | 11269 | 69.55 | 88.27 | 86.53 | 204.59 | 307.77 | 738.17 | 1166.89 | 2582.80 | 3679.77 | 6089.10 | | | | | | | |
| 02 | 11270 | 108.74 | 242.30 | 69.38 | 34.57 | 0.00 | 0.00 | 0.00 | 0.00 | 0.00 | 0.00 | 0.00 | 0.00 | 0.00 | 0.00 | 0.00 | 0.00 | 0.00 |
| 02 | 11276 | 155.75 | 255.32 | 322.67 | 193.87 | 97.11 | 66.56 | 0.00 | 0.00 | 93.66 | 176.81 | 298.47 | 508.68 | 600.08 | 1040.07 | 1845.48 | 2366.75 | 2736.56 |
| 02 | 11288 | 95.64 | 192.09 | 170.04 | 183.64 | 407.20 | 571.17 | 895.93 | 1662.92 | 2679.55 | 4223.34 | | | | | | | |
| 03 | 11237 | 116.72 | 115.78 | 45.94 | 12.65 | 0.00 | 0.00 | 0.00 | 0.00 | 0.00 | 0.00 | 0.00 | 0.00 | 0.00 | 0.00 | 0.00 | 0.00 | 0.00 |
| 03 | 11244 | 233.44 | 256.00 | 101.65 | 36.89 | 11.97 | 0.00 | 0.00 | 0.00 | 0.00 | 0.00 | 0.00 | 0.00 | 0.00 | 0.00 | 0.00 | 0.00 | 0.00 |
| 03 | 11245 | 110.01 | 165.51 | 349.87 | 389.56 | 602.01 | 1450.15 | 1927.01 | 4714.50 | 7447.65 | | | | | | | | |
| 03 | 11247 | 168.78 | 263.15 | 172.85 | 50.65 | 25.35 | 0.00 | 0.00 | 0.00 | 0.00 | 0.00 | 0.00 | 0.00 | 0.00 | 0.00 | 0.00 | 0.00 | 0.00 |
| 03 | 11264 | 150.08 | 194.36 | 148.77 | 151.22 | 0.00 | 0.00 | 0.00 | 0.00 | 0.00 | 0.00 | 0.00 | 0.00 | 0.00 | 0.00 | 0.00 | 0.00 | 0.00 |
| 03 | 11265 | 121.96 | 141.16 | 236.14 | 289.38 | 374.76 | 962.20 | 1562.71 | 2562.71 | 3682.44 | 4901.56 | | | | | | | |
| 03 | 11281 | 75.20 | 90.90 | 36.06 | 0.00 | 0.00 | 0.00 | 0.00 | 0.00 | 0.00 | 0.00 | 0.00 | 0.00 | 0.00 | 0.00 | 0.00 | 0.00 | 0.00 |
| 03 | 11289 | 93.41 | 109.80 | 73.81 | 66.41 | 55.68 | 127.42 | 205.34 | 340.20 | 604.39 | 1050.03 | 1480.22 | 1917.34 | 2547.62 | 4278.69 | | | |
| 04 | 11239 | 105.92 | 130.21 | 81.11 | 42.17 | 0.00 | 0.00 | 0.00 | 0.00 | 0.00 | 0.00 | 0.00 | 0.00 | 0.00 | 0.00 | 0.00 | 0.00 | 0.00 |
| 04 | 11249 | 131.09 | 245.40 | 328.17 | 223.28 | 177.06 | 357.63 | 455.97 | 910.09 | 1640.19 | 2614.29 | 3839.06 | | | | | | |
| 04 | 11251 | 210.58 | 295.98 | 669.19 | 683.19 | 986.53 | 2054.54 | 3341.94 | | | | | | | | | | |
| 04 | 11254 | 177.51 | 210.00 | 135.76 | 104.35 | 380.87 | 586.87 | 1149.45 | 2010.34 | 2455.90 | 4046.48 | | | | | | | |
| 04 | 11267 | 145.05 | 246.21 | 110.43 | 42.45 | 17.72 | 0.00 | 0.00 | 0.00 | 0.00 | 0.00 | 0.00 | 0.00 | 0.00 | 0.00 | 0.00 | 0.00 | 0.00 |
| 04 | 11272 | 90.66 | 135.37 | 103.80 | 48.45 | 26.57 | 22.81 | 22.54 | 0.00 | 0.00 | 0.00 | 0.00 | 0.00 | 0.00 | 0.00 | 0.00 | 0.00 | 0.00 |
| 04 | 11277 | 118.78 | 124.35 | 51.15 | 28.47 | 0.00 | 0.00 | 0.00 | 0.00 | 0.00 | 0.00 | 0.00 | 0.00 | 0.00 | 0.00 | 0.00 | 0.00 | 0.00 |
| 04 | 11280 | 93.70 | 184.72 | 101.03 | 32.53 | 0.00 | 0.00 | 0.00 | 0.00 | 0.00 | 0.00 | 0.00 | 0.00 | 0.00 | 0.00 | 0.00 | 0.00 | 0.00 |

The H8L2 antibody (in respective dosages of 5 mg/kg and 10 mg/kg) shows statistically significant anti-tumor effect on the PD-1 HuGEMM MC38-bearing mouse, which is more effective in tumor complete regression compared to the Keytruda 10 mg/kg treatment group.

### Industrial applicability

The method for increasing binding affinity of an IgG-like antibody to FcRn and prolonging serum half-life thereof according to the present disclosure is capable of effectively increasing binding affinity to FcRn of the IgG-like antibody and prolonging serum half-life of the IgG-like antibody, while the modified IgG-like antibody produced exhibits unchanged binding affinity to a specific antigen.

Although embodiments of the present disclosure have been described in detail, it will be understood by those skilled in the art that various modifications and substitutions can be made in these embodiments as the teaching disclosed, and such the changes are all within the scope of the present disclosure which is given by the appended claims and any equivalents thereof.

In the specification of the present disclosure, the terms "an embodiment", "some embodiments", "a specific embodiment", "an example", "a specific example", "some examples" and the like are intended to refer to particular features, structures, materials or characteristics described by way of example or embodiment are contained in at least one embodiment or example of the disclosure. In this specification, the schematic representation of the above terms does not necessarily refer to the same embodiment or example. Moreover, the particular features, structures, materials or characteristics described may be combined in any suitable manner in one or more embodiments or examples.

## Claims

1. A method for increasing binding affinity of an IgG-like antibody to FcRn and prolonging serum half-life thereof, comprising:
mutating amino acids at positions 254, 308 and 434 in an FcRn-binding site of a heavy chain constant region of the IgG-like antibody.

2. The method according to claim 1, wherein the amino acids at positions 254, 308 and 434 in the FcRn-binding site of the heavy chain constant region of the IgG-like antibody are mutated into threonine, proline and alanine respectively.

3. A modified IgG-like antibody, wherein the modified IgG-like antibody has amino acid mutations at positions 254, 308 and 434 in an FcRn-binding site of a heavy chain constant region with respect to a wild-type IgG-like antibody.

4. The modified IgG-like antibody according to claim 3, wherein the amino acid mutations at positions 254, 308 and 434 in the FcRn-binding site of the heavy chain constant region of the modified IgG-like antibody are respectively threonine, proline and alanine with respect to the wild-type IgG-like antibody.

5. A method for preparing the modified IgG-like antibody according to claim 3 or 4, comprising:
generating a nucleic acid sequence encoding a target IgG-like antibody via gene synthesis according to an amino acid sequence of the target IgG-like antibody;
constructing an expression vector comprising the nucleic acid sequence encoding the target IgG-like antibody; and
transfecting an antibody producing cell with the expression vector, such that the antibody producing cell expresses and secretes the modified IgG-like antibody.

6. The method according to claim 5, wherein the antibody producing cell is a 293 cell.
